# EUROPEAN PATENT APPLICATION

(11) **EP 4 261 833 A1**
(43) Date of publication of application: **18.10.2023**
(21) Application number: 22167699.2
(22) Date of filing: 11.04.2022
(51) Int. Cl.: G16H 20/17, G16H 40/63

(54) **GUIDANCE FOR A DRUG DELIVERY PROCESS**

(71) Applicant: Ypsomed AG, 3401 Burgdorf (CH)
(72) Inventor: Schmid, Christian, 5000 Aarau (CH); Bosshard, Simon Martin, 3324 Hindelbank (CH)
(74) Representative: Kleiner, Stefan

(57) **Abstract**

The present invention relates to a drug delivery guidance system including a drug delivery device (1) for dispensing a liquid drug from a reservoir of the delivery device (1). The guidance system includes wearable glasses (20) providing a user field of view and adapted to display information in an overlay (24) in the user field of view and an electronic module (3) integrated in or releasably attachable to the drug delivery device (1), comprising delivery status sensing means for monitoring a delivery status parameter. The electronic module (3) further includes a communication unit adapted to communicate a sensed delivery status parameter value to the wearable glasses (20), wherein the glasses (20) are adapted to display in the overlay (24) status information (23, 26) based on the parameter value.

## Description

### FIELD OF THE INVENTION

The present invention relates to medicament delivery devices for injecting, delivering, administering, infusing or dispensing substances and/or liquids such as insulin, hormone preparations or vaccines. It departs from a drug delivery guidance system including a drug delivery device, wearable glasses and an electronic module integrated in or releasably attachable to the drug delivery device. The electronic module includes delivery status sensing means for monitoring and sensing a delivery status parameter.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the injection pen. Alternatively, in a predefined fix dose may be set and the user can administer the fix dose. A dose is dispensed by inserting the needle into a suited portion of human skin and by moving the piston manually or by pressing a release button of an automatic injection device. Automatic injection devices may comprise an electronic dose dial mechanism to automatically set a dose.

Drug delivery device based therapies generally benefit from an electronic module or electronic unit embedded or integrated in the delivery device, or being part of an auxiliary or supplemental electronic module or add-on device releasably attachable to the delivery device. The electronic module monitors a drug delivery process, in order to proactively prevent false handling of the device and/or to keep track of the doses already applied, and generates data related to an instantaneous condition and/or use of the delivery device.

Several approaches exist to use these data of the electronic module to support a user or guide the user through a delivery process with the drug delivery device. A known approach is to guide the user by means of augmented reality (AR) application and devices which provide a combination of real word objects and virtual objects. The latter are usually displayed in an overlay in a user field of view.

Document US 9,832,412 B2 discloses a system that operates in a hands-free manner using glasses with a camera and an augmented reality display. The glasses are configured to identify and recognize a syringe by means of a camera. The syringe includes an identification tag such as a QR code. The identification tag provides information about the syringe and the fluid contained therein such as a medication type, total fluid volume, manufacturer, needle dimensions and a fluid expiration date. Depending on its position the identification tag can be used to estimate the position of the plunger rod of the syringe. An image processing software can record the initial position of the plunger rod relative to the position of the identification tag. When the plunger rod moves relative to the position of the identification tag, the image processing software determines that an injection has begun. The detected information can be displayed on a virtual layer projected over the user's field of view in the display of the glasses. Furthermore, the virtual layer can assist the user in positioning the syringe by providing a virtual trace of the location of a vein suitable for needle insertion.

US2020051244 A1 discloses a user guidance with a mobile device. An image of the injection pen and a titration level indicator are displayed on a display of the mobile device. A camera of the mobile device captures a titration level indicator of the injection pen and provides an indication of the determined level and displays comments to the user.

WO19096875 A1 discloses a system for training and assisting of a user of an auto-injector device via augmented reality. The augmented reality application is embedded in a smartphone to display overlays in a smartphone screen or lenses of an optical head-mounted display. After identifying the injection device by camera data of a smartphone camera the software generates overlays on the smartphone display. The overlays include boxes containing textual information on the use of certain parts of the injection device and a textual information with the injection device's identification code. If a time or date differs from a prescribed medication plan, the content, color, size or position of the overlay alters such that the user can easily recognize the overlay and note that an injection is actually not required and prescribed.

These prior art approaches exclusively rely on camera data originating from a camera capturing a visible appearance of the delivery device. Consequently, the augmented reality application derives a current user situation or delivery status or condition based on the camera data. The determined status thus strongly depends on the quality of the camera data.

### DESCRIPTION OF THE INVENTION

It is an objective of the invention to improve the guidance of a user through a drug delivery process by means of reliable information accessible in a hands-free manner.

This objective is achieved by a drug delivery guidance system, a computer program product or a method according to the independent claims. Preferred embodiments are evident from the dependent claims.

The invention relates to a drug delivery guidance system including a drug delivery device for dispensing a liquid drug from a container, reservoir or syringe of the delivery device. The guidance system further includes wearable (head-worn) glasses providing a user field of view by means of lenses or glasses. The glasses are adapted to display information on a visual overlay in the user field of view. Furthermore, the guidance system includes an electronic module integrated in the drug delivery device or releasably attachable to a housing of the drug delivery device. The electronic module comprises delivery status sensing means for sensing or monitoring at least a (current) delivery status parameter. The electronic module includes further a communication unit adapted to communicate a sensed and determined delivery status parameter value to the wearable glasses (directly or indirectly via intermediate device) by means of a wireless data connection to the glasses. The glasses are configured to display in the overlay delivery status information based on the received delivery status parameter value.

Prior art approaches focus on wearable glasses with a camera that captures the drug delivery device and usually identifies a marker or code on an outside of the delivery device or a packaging thereof. The glasses are usually adapted to display information in an overlay or visual layer in a camera preview provided by the glasses or a mobile device such that the user experiences an augmented reality. However, such optical capturing is limited to what a camera or optical detecting means are capable to detect and imaging software is capable of deriving from the camera data.

The guidance system for a drug delivery process according to the invention provides reliable communication between an electronic module integrated in a delivery device or releasably attached to a drug delivery device and wearable augmented reality glasses (AR glasses). For the system according to the invention a camera is not required as the data originate from delivery status sensing means in the electronic module which directly senses or measures a delivery status parameter during the delivery process with the drug delivery device. That means the electronic module may provide precise and current sensed information such as, for example, a set dose, a delivered dose, a drug temperature or a delivery device battery status. Such a system requires sensing means in the electronic module but on the other hand the electronic module provides reliable delivery status data determined in close proximity of the delivery mechanism of the delivery device. Such a sensing exceeds by far the capabilities of known optical capturing systems.

According to the invention the wearable glasses can display in an overlay delivery status information based on or originating from a delivery status parameter value, preferably directly from the electronic module. When wearing the glasses the user thus can not only see the delivery device through the glasses but also the status information on the visual overlay. Hence, the user is therefore guided in a hand-free manner by augmented reality and can operate the delivery device with his or her hands.

The electronic module may be embedded in or an integral part of the delivery device, e.g. the electronic module is inside a housing of the delivery device. In this case the delivery device is, for example, an smart injection pen (with or without motor) or an electronic drug pump. Alternatively, the electronic module may be implemented as separate module having a module housing and being adapted to be releasably attached to a housing of the drug delivery device, for example, by a clamping mechanism or a thread. Such separate electronic modules are also named as add-on, auxiliary device or supplemental device. Hence, in this case the electronic module can be used for different drug delivery devices as the electronic module can be detached from one delivery device and re-attached to another delivery device.

In both cases, the electronic module includes status sensing means for sensing or monitoring a current delivery status parameter of a delivery process performed by a delivery mechanism of the delivery device. The sensing means may be, for example, a sensor adapted to detect a set dose or a dispensed dose, a current or voltage meter or a control circuit for detecting a device status of the delivery device or a reader for capturing information of a tag or code of a currently present drug in the delivery device.

The term "delivery process" used herein should be understood to include the whole process of delivery of a drug into the user. That means the "delivery process" encompasses also steps just before the actual delivery such as preparation steps or a priming process and the term includes also steps just after the delivery such as a holding period right after dispensing of the drug.

The electronic module comprises preferably processing means or a controller, for example, a microcontroller or FPGA for running the guidance system according to the invention. The electronic module preferably comprises a data storage unit for storing determined or calculated data or/and a sensor signal from the sensing means.

The drug delivery device may be a disposable injection pen comprising a reservoir (cartridge) with the liquid drug, a semi-disposable injection pen with a reusable drive unit and a replaceable receptacle including a reservoir, a reusable injection pen with replaceable reservoir (cartridge) or an autoinjector comprising a syringe. The drug delivery device preferably includes a plunger rod to dispense the drug from the reservoir.

The delivery device may be further a patch injector applicable onto the skin of the user by means of an adhesive layer for the duration of the injection. The delivery status parameter value may be a real-time or instantaneous fill-level of the reservoir, or any other dynamic or continuous drug dispense progress indicator. Displaying such a progress indicator is particularly helpful in case a reservoir window of the delivery device is not permanently visible, because e.g. covered by clothing. Alternatively, the drug delivery device may be an infusion system, for example, a conventional medical drug pump such as an insulin pump with tubing or it may be a drug patch pump without tubing that is attachable directly onto the skin of the user.

The term "injection system" or "injector" refers to a device that is removed from the injection site after each medication event or drug delivery process, whereas the term "infusion system" or "infusion device" refers to a device with a cannula or needle that remains in the skin of the patient for a prolonged period of time, for example, several hours.

The head-worn glasses feature augmented reality (AR) capabilities, namely the glasses provide a user field of view through the glasses or lenses and are adapted to display information on a visual overlay in the field of view. In other words, the wearable glasses provide a virtual layer showing the status information and projected over the user field of view. That means when wearing the glasses the user sees the displayed information in the field of view. Such glasses are also named as smart glasses or augmented reality glasses or AR glasses. The glasses comprise thus an electronic circuit or processing means to process the received delivery status parameter value and to generate display data to display the status information. Further, the glasses include communication means allowing at least to receive wirelessly data from an external device, preferably from the electronic module or from an interconnected device. Examples for such communication means are near field communication (NFC), Bluetooth or a mobile communication network. Further electronics such as enhanced computing means, loudspeakers or a camera may be included in the glasses although not required by the system according to the invention.

In the present description the plural form glasses is used. However, the term encompasses any type of AR eyewear including a monocle, a single glass or a glass part of a helmet featuring AR capabilities.

At least one and preferably a plurality of delivery status parameter values is/are determined, monitored, read or sensed by the sensing means of the electronic module before, during or after a delivery process performed by the drug delivery device. Hence, the status parameter value is a value that is actually derivable from an element or mechanism of delivery device. As mentioned above examples of the status parameter are a set dose, a delivered dose, a drug temperature or a delivery device battery status.

The status information displayed by the wearable glasses are based on at least one sensed status parameter value. That means the status information may just include a sensed status parameter value or a parameter value may be processed prior displaying by processing means in the glasses to generate the status information for the user. For example, the status parameter value may be displayed alphanumerically or used in a visualization or chart to simplify a displaying or it may be set in relation to another value. In any case, the status information are based on (or at least partially originate from) the sensed status parameter value form the electronic module.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

In a preferred embodiment the sensed status parameter value is an instantaneous value which may change over time during the delivery process. The electronic module is preferably adapted to communicate in real time or quasi real time the sensed instantaneous parameter value during a delivery process and the wearable glasses are preferably adapted to update in real time or quasi real time the displayed status information based on the status parameter value.

The electronic module may periodically sense or measure instantaneous status parameter values just before, during or directly after a delivery process. Thus, the user is provided with current status information during the whole drug delivery process.

The sensed status parameter values are preferably transmitted to the glasses by a direct wireless data connection between the electronic module and the glasses. Thus, the user is continuously provided with updated status information. Such updated information, for example, a progress indication of the entire injection or infusion procedure may help to guide the user through the drug delivery process.

Preferably, one of the at least one status parameter value sensed by the sensing means of the electronic module is a delivery mechanism parameter of the delivery device such as set dose value, a dispensed dose value, a corrected dose value, an elapsed dispensing time, an instantaneous fill level value of the reservoir in the drug delivery device. Furthermore, the parameter value may be an elapsed holding time, a temperature value, a priming status or a power source level value of the delivery device, a connection state between the electronic module and the delivery device or a verification status of the drug. The latter may be determined by the electronic module if the sensing means read drug information from a tag or code of the drug and if the read information are sent to an external database which can verify the drug information and can send a notification back to the electronic module.

As mentioned above the status parameter value can be a measured value not only during the actual delivery but also just before or after the delivery process.

The status information generated by the glasses based on the received status parameter value and displayed in the overlay may include in a preferred embodiment a visualization of a relationship between the sensed status parameter value and a predefined reference value. If a plurality of status parameter values are sensed for each of the parameter values a reference value may exist. The relationship may help the user to capture the information and over all may help to see a current status in an overall context.

The reference value may be a non-variable maximum, minimum, target or predefined value. The reference value is preferably stored in the electronic module or received by the electronic module prior a comparison with the sensed status parameter value.

The visualization may help and guide the user through the drug delivery process. Namely, the visualized relationship between the sensed parameter value and the reference value may indicate a level of progress, for example, a remaining time or remaining amount of drug to be administered. The visualization thus may help to inform the user in a simple manner and may avoid an excessive demand placed on the user for reading information.

Preferably, the visualization comprises a progress indicator, for example, a straight or linear progress bar, status bar, a curved or a multidimensional progress cycle or an updated pie chart. Such indicators allow a quick and easy visualization of the relationship between the current sensed status parameter value and the reference value. Furthermore, such a visualization is in particular an advantageous for a simple indication of data without requesting the user to read figures.

Alternatively, the visualization may include a coloring of figures and text to facilitate the reading of the displayed process information.

The reference value is preferably a fix value which does not alter during the delivery process and which is defined previously. The reference value is preferably one of a maximum dose value, a total holding time, a total capacity value of the reservoir, a maximum or minimum temperature value or a maximum or minimum power level value.

In a further preferred embodiment the wearable glasses comprise a camera, an optical sensor or optical detecting means to capture the drug delivery device such as a photosensor or an infrared camera. The camera may be, for example, arranged on a frame or on side pieces of the wearable glasses. The glasses are adapted to provide a camera preview of the camera or the optical sensor in the user field of view such that the user may see the delivery device through the preview and the overlay with the status information at the same time. Hence, based on the camera data (e. g. image data, infrared data), the glasses can optimally position the status information in the field of view relative to an image of the captured delivery device. That may help to optimize the information intake for the user.

The glasses may dynamically arrange the status information during the delivery process. That means the displayed status information and its position within the user field of view may vary over time during the delivery process, in particular if the user moves the drug delivery device and the head-worn glasses relative to each other.

Alternatively, in absence of the camera the glasses may arrange the status information in a fixed position, for example, near the bottom, at the top or at the edge of the user field of view of the wearable glasses.

In a preferred embodiment the guiding system provides a verification for the sensed status parameter value with an optical detected status or condition by means of the camera. That means the glasses receive not only the status parameter value from the electronic module but have also access to camera data from the camera. That allows to verify the sensed status parameter value with captured optical data or vis versa. For example, a drug fill level determined by the electronic module can by verified and confirmed by the camera data if the camera captures the delivery device and thus captures from outside a fill level of the drug in the reservoir.

Such a redundancy may be advantageous if a status parameter value is of particular importance such as a completed delivery process, a crucial holding time or a remaining amount of drug inside a reservoir of the delivery device.

The glasses may preferably comprise an electronic circuit or controller including a software application configured to identify or derive a delivery process condition or status from the camera data for the comparison. If the sensed status parameter value is not in accordance (not within a predefined range or exceeds a threshold) with the derived status from the camera data the glasses may notify the user accordingly. Optionally, the glasses may also notify the user in case the status parameter value matches to or is in accordance with the derived status from the camera data.

The communication unit of the electronic module is preferably connected by a wireless communication with a corresponding communication circuit in the wearable glasses. The communication may be performed, for example, by a wireless local area network (WLAN) or by another short or near range wireless communication technology such as Bluetooth, ZigBee, WiMAX or NFC.

Such a direct communication provides an immediate transmission of sensed status parameter values from the electronic module to the glasses or any other data transmission between the electronic module and the wearable glasses.

Alternatively, the communication unit of the electronic module may be connected to an intermediate device (e.g. a getaway device) which in turn is connected to the wearable glasses. The data from the electronic module are then transmitted indirectly to the glasses.

The communication unit of the electronic module and the communication means of the glasses are preferably configured to communicate with an external cloud server. Therefore, the sensed status parameter value may be transferred to the cloud server by the communication unit of the electronic module and the glasses may communicate with the cloud server and receive the parameter value and optionally additional information from the cloud server.

The cloud server may be an external database. By way of example, before the sensed status parameter value is transferred to the glasses the parameter value may be approved, verified or registered by a healthcare practitioner, a healthcare insurance or a manufacturer of the drug or the drug delivery device via data transmission to the cloud server. Therefore, enhanced and approved data may be provided to the user via external cloud server.

In a preferred embodiment the electronic module and preferably also the drug delivery device are devoid of any electronic indicating or display means other than a viewing window for the user. That means the electronic module and the drug delivery device do not comprise any user interaction elements such as a display, LED, lamps or the like. All information or indications from the delivery device are transmitted to the user by the status information in the overlay displayed by the wearable glasses.

That simplifies the electronic module and the drug delivery device as all user interaction is exclusively provided by the glasses.

The glasses are preferably configured to determine and to display an operating step instruction for operating the drug delivery device based on the status parameter value received from the electronic module. The operating step may include, for example, an indication to the user how to set a dose (e. g. direction of rotation of a dose knob of the delivery device), how to perform a priming, how to decap or prepare the device for drug delivery or how to set correctly the device onto the injection or infusion site.

The operating step instruction is displayed by the worn glasses in the overlay and thus the user can see the instruction and at the same time can use the hands for operating the delivery device. The operating step instruction can be, for example, a text message, a sign or any graphic representation.

In case the glasses include a camera the operating steps may be displayed in an overlay in a camera preview of the camera. This allows to mark parts of the image of the captured delivery device in the overlay (e. g. a colouring of a dose button of the delivery device) and thus allows to facilitate the indication for the user.

The drug delivery device is preferably an injection device and in particular a reusable or disposable injection pen. The injection device may be manually operated by the user to dispense a dose from the reservoir or the injection device may alternatively include automatic drive means such as a pre-tensioned drive spring. In a preferred embodiment the delivery device is an autoinjector comprising a housing which encases a drive mechanism with a pretension spring and the electronic module. In this case the electronic module is integrated in the injection device.

Alternatively, the drug delivery device is an electronic drug pump.

The invention relates further to a computer program product comprising instructions which, when executed by a computing device in wearable glasses, cause the computing device to carry out the steps of
a. Establishing a data communication between the wearable glasses and an electronic module integrated in or releasably attached to a drug delivery device;
b. Receiving a delivery status parameter value from the electronic module;
c. Generating display data including status information for a user based on the received status parameter value;
d. Providing the display data to display means of the wearable glasses to display the status information in an overlay in a user field of view of the wearable glasses.

The computing device is preferably inside a housing of the wearable glasses. By way of example the computing device may be a controller e.g. a microprocessor or FPGA inside, for example, a front or side pieces of the wearable glasses.

Furthermore, the invention relates to a method for guiding a user through a drug delivery process with a drug delivery device, the method comprising the steps of
a. Sensing, by an electronic module integrated in or releasably attached to the drug delivery device, a delivery status parameter value;
b. Transmitting, by the electronic module, the sensed status parameter value to wearable glasses;
c. Processing, by a computing device in the glasses, the parameter value and generating status information for a user based on the status parameter value;
d) Displaying, by the glasses, the status information in an overlay in a user field of view of the wearable glasses.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a drug delivery guidance system according to the invention with an electronic autoinjector and AR glasses;
- Fig. 2: depicts a second embodiment of the guidance system with an add-on attached to an autoinjector;
- Fig. 3: depicts a third embodiment comprising a patch pump and
- Fig. 4: depicts a forth embodiment including an injection pen, wherein dose setting is guided by the AR glasses.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

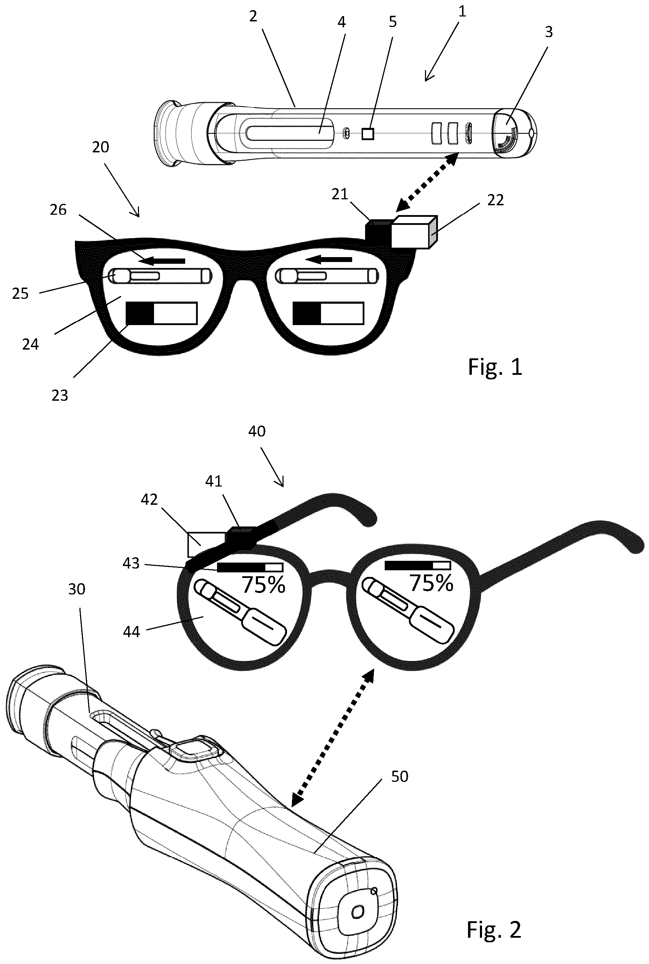

Figure 1 depicts a first embodiment of a drug delivery guidance system according to the invention. The guidance system includes an autoinjector 1 with an integrated electronic module 3 and wearable augmented reality glasses 20 (AR glasses) including a communication unit, a camera 22 and processing means 21.

In the following the features of the autoinjector 1 and the AR glasses 20 are described in detail. Subsequently, the function of the guidance system will be explained.

The autoinjector 1 includes a dispensing mechanism with a pre-tensioned spring, a reservoir in form of a syringe (all not shown), an electronic module 3 and a housing 2 which encloses the syringe, the dispensing mechanism and the electronic module. The pre-tensioned spring is adapted to dispense a liquid drug from the syringe by driving a plunger rod in a distal direction thereby moving a piston inside the syringe in dispensing direction to dispense the drug from the syringe through an injection needle (not shown) into the user.

The electronic module 3 inside the housing 2 of the autoinjector 1 includes a controller, sensing means and a communication unit. The sensing means include a sensor to determine delivery status parameter values in form of a position sensor adapted to determine the position of the plunger rod. Hence, based on the position sensor signal the electronic module 3 can determine if the autoinjector 1 was used and whether the drug was fully dispensed from the syringe. The sensing means further include a drug temperature sensor and a sensor to read drug information (not shown) on an outside of the syringe. Such information includes the type and expire date of drug. The communication unit is configured to establish a wireless data communication (depicted as dotted line) to the AR glasses 20 to exchange data and in particular to send the measured delivery status parameter values from the autoinjector 1 to the AR glasses 20.

The communication unit can additionally be connected to an external cloud server (not shown) and thus the measured parameter values may be additionally sent to the cloud server.

The AR glasses 20 include a frame with glasses or lenses and side pieces such that the AR glasses can be head-worn by a user. In addition to common glasses the AR glasses 20 include an AR display to generate an AR overlay 24 in a user field of view, processing means in form of a microcontroller 21, a communication means and the camera 22. The AR display is configured to generate the overlay 24 with status information visible for the user in the user field of view. That means the overlay 24 is a virtual layer projected on the lenses such that the user can see and read the status information provided by the glasses 20. Hence, the user experiences an augmented reality based on the real view through the lenses and the virtual overlay 24 with the displayed status information.

The AR glasses 20 includes the communication means for a data communication with the electronic module 3 of the autoinjector 1. The latter can be captured by the camera 22 of the glasses to generate camera data just before, during and after the delivery process.

As marked with the dotted line in figure 1 the electronic module 3 of the autoinjector 1 and the AR glasses 20 are connected by a wireless data communication in form of a near field communication (NFC) or Bluetooth connection. The communication unit of the electronic module 3 and the AR glasses are further configured to be connected to a user mobile device (not shown) such as a mobile phone. This may be used, for example, for the pairing process. The mobile device can further be used to receive the data that are transmitted form the autoinjector 1 to the AR glasses 20.

When using the guidance system according to the invention the user wears the AR glasses 20 and orients the view to the autoinjector 1 and the injection site, respectively. As illustrated in figure 1 the user sees in the field of view not only an image 25 of the autoinjector but also the overlay 24 provided by the AR glasses 20. The overlay 24 provides status information which are based on instantaneous delivery status parameter values measured and transmitted to the AR glasses 20 by the electronic module 3 in the autoinjector 1.

An exemplary embodiment with a periodically updated fill level is descripted in detail. As mentioned above other status parameters are determined by the electronic module 3 and are sent to the AR glasses 20 for displaying in the AR overlay 24.

The electronic module 3 measures a start and an end position or periodically measures the plunger rod position by means of the position sensor. Based on the position signal the electronic module can determine the amount of dose already dispensed or/and the remaining amount of drug inside the syringe. The electronic module periodically sends an instantaneous fill level value to the AR glasses 20. The processing means 21 of the AR glasses 20 generate subsequently a user visualization which is displayed on the AR layer 24.

The visualization aims to provide current and easy viewable status information from the ongoing injection process to the user. That is, the instantaneous fill level or the instantaneous dispensed amount of the dose is set in relation to a total amount of dose or a total capacity of the syringe. Such a relationship is displayed as a progress bar 23 next to the camera preview image 25 of the autoinjector. Figure 1 shows an arrangement where the progress bar 23 includes a black (filled) and a white (empty) part. The black part shows the remaining amount of the dose to be administered and the white part shows the already dispensed amount of the dose. The progress bar 23 is periodically updated such that the user is provided with updated injection progress information. Additionally, the injection direction is shown by an arrow 26 positioned on the top in the user field of view.

Once the injection is completed and hence the position sensor of the electronic module 3 detects an end position of the plunger rod a corresponding notification is sent to the AR glasses 20 and displayed in the overlay 24 to the user. The user is thus informed when the total amount of drug is administered. The AR glasses may subsequently display a down counter for a holding time of several seconds during that time the user has to hold the autoinjector onto the injection site to ensure full absorption of the drug.

As mentioned above the AR glasses 20 include a camera 22 and provide a corresponding camera preview in the user field of view. The AR overlay 24 is projected on the camera preview. Thus, when the user wears the AR glasses 20 he or she sees the camera preview and the displayed AR information in the user field of view. The camera 22 captures the autoinjector 1 which includes a marker 5. Based on the captured marker 5 in form of a QR code an image processing software running by the processing means 21 of the AR glasses 20 can identify the position of the autoinjector 1 and is configured to arrange dynamically the progress bar 23 and the arrow 26 in the field of view relative to the image 25 of the captured autoinjector. The marker 5 includes additionally information about the autoinjector and/or the drug inside. After capturing these information the AR glasses 20 can display these data to the user via AR overlay 24.

Additionally, based on the camera data the AR glasses 20 are configured to display in the overlay 24 operating instruction to the user. That is, a dispensing button of the autoinjector 1 is colored in the image 25 of the autoinjector in the camera preview to guide the user through the dose setting process.

The software of the AR glasses 20 further uses the camera data to verify the sensed parameter values from the electronic module. That means a determined filled level of the drug in the autoinjector 1 can be verified as the autoinjector 1 includes an opening or window 4 in the housing 2 allowing the camera (and the user) to capture a current fill level of the syringe. Further a spatial orientation of the autoinjector 1 is captured by the camera 22. Subsequently, the image processing software in the AR glasses processes the camera data, determines a fill level based thereon and compares the determined fill level with the received fill level value from the electronic module. The AR glasses can thus verify the received parameter value and display a corresponding notification to the user.

Figure 2 depicts a second embodiment of the present invention. The system includes a disposable injection device. Figure 2 shows a common autoinjector but any injection device such as a reusable or disposable pen device can be used for the guidance system as described herein.

In contrast to the first embodiment in the embodiment according to figure 2 the electronic module is a separate add-on 50, which is releasably attached to the injection pen 30. The add-on 50 is adapted to monitor an injection process performed by the injection pen 30, if the add-on is attached to the pen 30. For that purpose, the add-on 50 features sensors as described with respect to the first embodiment. By way of example, the add-on 50 includes an induction sensor adapted to sense a position of a drive spring (not shown) inside a housing of the injection pen 30. During dispensing the (metallic) drive spring moves and causes a change of a magnetic field which in turn can be detected by the induction sensor in the add-on 50. The add-on 50 comprises additionally a temperature sensor, a battery status sensor and a RF sensor (all not shown) to read an RFID tag on the injection pen 30.

Thus, the add-on 50 provides measured parameter values including a plunger rod position or a determined dispensed amount of dose or remaining amount of dose or a priming state (from the plunger rod position sensor), a drug temperature value (from the temperature sensor), or drug information (from the RF sensor). As descripted with respect to the first embodiment the sensed parameter values are transmitted from the electronic module to the AR glasses 40.

As in the first embodiment the AR glasses 40 include a camera 42 and an AR overlay 44 is arranged on a camera preview of the camera 42. The visualization generated by processing means 41 in the second embodiment includes a progress bar 43 indicating a remaining amount of drug in syringe in the injection pen 30 in relation to a total capacity of the syringe. Next to the progress bar 43 a percentage figure is displayed. The progress bar is positioned next to the image of the captured injection pen in the user field of view such that both the injection pen 30 with the add-on 50 and the progress bar 43 are optimally visible for the user.

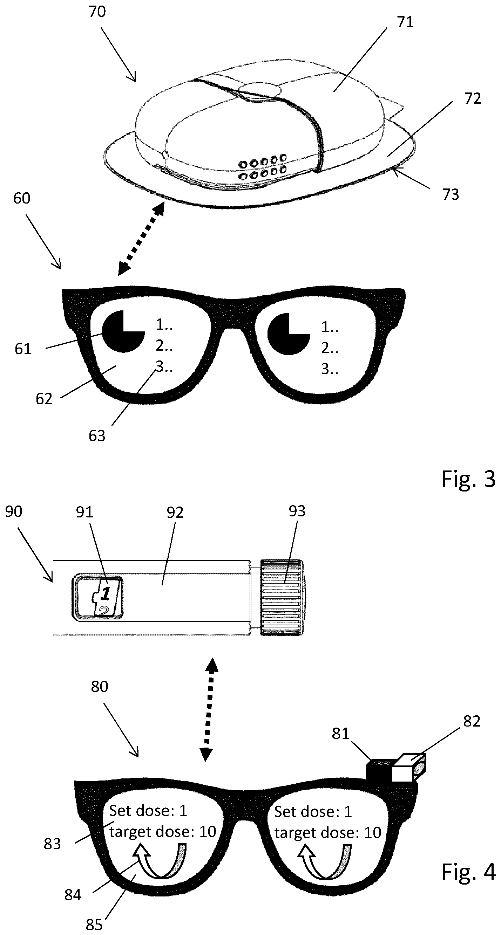

Figure 3 depicts a third embodiment of the guidance system according to the invention including a patch pump 70 adapted to be applied directly onto the skin of the user. The guidance system in this embodiment guides the user through the preparation process for installing the patch pump 70 onto the body of the user.

The patch pump is devoid of any user elements and hence does not comprise any LED, display or the like. That means all information for the user is shown to the user by the AR display in the AR glasses 60.

The electronic module (not shown) is integrated in the patch pump 70 and includes sensors as descripted with respect to the first embodiment. In addition, the electronic module includes in this embodiment an orientation sensor and a cover layer sensor (not shown) configured to determine whether a cover layer 73 of the adhesive layer 72 is present or was removed from the adhesive layer 72. That means the electronic module is additionally capable of providing a parameter values representing a preparation condition of the patch pump 70. The AR glasses 60 can thus display a preparation progress or current preparation state on the AR overlay 62.

As shown in figure 3 the progress is displayed by means of a pie chart 61 and instruction steps 63 in short text displayed next to the chart 61. Thus, the AR glasses 60 display status information based on the layer sensor data and orientation sensor data. This can be, for example, a confirmation to the user that the initial present cover layer 73 was removed correctly and/or that the patch pump 70 is mounted correctly onto the user body. The latter may be determined by the orientation sensor as this sensor is configured to sense the spatial orientation of the patch pump 70.

In the third embodiment according to figure 3 the AR glasses 60 do not include any optical detection means or a camera. That means the AR glasses 60 exclusively receive the data from the electronic module in the patch pump 70 for the displaying via AR overlay 62.

Figure 4 shows a fourth embodiment of the guidance system with an electronic injection pen 90 including a manual dose dialing mechanism with a dose dialing sleeve 91 and a dose knob 93. To set a dose or to correct a dose the user can manually rotate the dose knob 93 relative to a pen housing 92 clockwise or counterclockwise, respectively. An electronic module (not shown) inside the pen housing 92 includes a rotational position sensor (not shown) configured to determine a current rotational position of the dose knob 93. Based on rotational sensor data the electronic module determines a current set dose or a dispensed amount dose. Subsequently and as described above, the electronic module transmits the dose data to the AR glasses 80.

Although figure 4 shows the dose dial sleeve 91 with circumferentially arranged dose numbers visible from outside such a numbering is not mandatory as the user is informed by an AR overlay 85 of the glasses 80 about the current set dose.

The AR glasses 80 guide the user through the dose setting procedure in that the AR glasses 80 display a current set dose in the AR overlay 85 in the user field of view. As shown in figure 4 the visualization includes a figure 83 representing a current set dose next to a target dose to be set. This predefined target dose originates from a user-specific therapy plan. The AR glasses 80 are thus wirelessly connected not only to the electronic module in the electronic injection pen 90 but also to a cloud server providing the user therapy plan. Hence, when wearing the AR glasses 80 and when setting a dose the user sees in the field of view the current set dose and the target dose. The visualization includes further an arrow 84 indicating the direction the dose knob 93 is currently turned.

In the fourth embodiment the AR glassed 80 do not include a camera but comprise a loudspeaker 82 and processing means 81 configured to generate audible output. The loudspeaker 82 indicates the current dose by an acoustic signal, e. g. by a voice that speaks the current dose. This is in particular advantageous for user with an impaired eyesight. In an alternative embodiment the audible output is directly transmitted to a user hearing aid or hearing device. The loudspeaker is thus not required.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

| | | | |
|---|---|---|---|
| 1 | Autoinj ector | | |
| 2 | Housing | 70 | Patch pump |
| 3 | Electronic module | 71 | housing |
| 4 | Window | 72 | adhesive layer |
| 5 | Marker | 73 | cover layer |
| 20, 40 | AR glasses | | |
| 21, 41 | Processing means | 80 | AR glasses |
| 22, 42 | Camera | 81 | Processing means |
| 23, 43 | progress bar | 82 | Loudspeaker |
| 24, 44 | AR overlay | 83 | Set dose and target dose |
| 25 | autoinjector in camera preview | 84 | Arrow |
| 26 | Arrow | 85 | AR overlay |
| 30 | Injection pen | 90 | Electronic pen |
| 50 | Add-on | 91 | dose sleeve |
| | | 92 | housing |
| 60 | AR glasses | 93 | dose knob |
| 61 | Pie diagram | | |
| 62 | AR overlay | | |
| 63 | Text information | | |

## Claims

1. A drug delivery guidance system including
- a drug delivery device (1) for dispensing a liquid drug from a reservoir of the delivery device (1);
- wearable glasses (20) providing a user field of view and adapted to display information in an overlay (24) in the user field of view;
- an electronic module (3) integrated in or releasably attachable to the drug delivery device (1), comprising sensing means for sensing at least one delivery status parameter,
**characterized in that**
the electronic module (3) includes a communication unit adapted to communicate a sensed delivery status parameter value to the wearable glasses (20),
wherein the glasses (20) are adapted to display in the overlay (24) status information (23, 26) based on the received delivery status parameter value.

2. Guidance system according to claim 1, wherein the delivery status parameter value is an instantaneous value and wherein the electronic module (3) is adapted to communicate in real time instantaneous values to the wearable glasses (20) during a delivery process and wherein the glasses (20) are adapted to update in real time the displayed status information (23, 26).

3. Guidance system according to claim 1 or 2, wherein the at least one delivery status parameter value is a set dose value, a dispensed dose value, a corrected dose value, an elapsed holding time, an elapsed dispensing time, an instantaneous fill level value of the reservoir, a temperature value, a priming status or a power source level value, a connection state of the communication unit or a verification status of the drug.

4. Guidance system according to any of claims 1 to 3, wherein the displayed status information (23) includes a visualization of a relationship between the at least one sensed status parameter value and a predefined reference value.

5. Guidance system according to claim 4, wherein the visualization includes a progress indicator (23), preferably a progress bar or a multidimensional progress cycle.

6. Guidance system according to claim 4 or 5, wherein the reference value is a maximum dose, a total holding time, a total capacity of the reservoir, a maximum or minimum temperature or a maximum or minimum power level.

7. Guidance system according to any of claims 1 to 6, wherein the wearable glasses (20) include a camera (22) to capture the drug delivery device (1) and wherein the user field of view is a camera preview of the camera (22) and wherein the glasses (20) are adapted to arrange the overlay in the camera preview.

8. Guidance system according to claim 7, wherein the glasses (20) are configured to determine a delivery state of the drug delivery device (1) based on camera data from the camera (22) and are configured to compare the determined delivery state with the status parameter value and wherein the glasses (20) are further configured to display a message to the user based on the comparison.

9. Guidance system according to any of claims 1 to 8, wherein the glasses (20) are directly connected to the electronic module (3) by a wireless data communication.

10. Guidance system according to any of claims 1 to 9 further comprising a cloud server, wherein the communication unit is additionally configured to communicate with the cloud server and wherein the glasses are configured to communicate with the cloud server.

11. Guidance system according to any of claims 1 to 10, wherein the electronic module (3) and preferably the drug delivery device (1) are devoid of any electronic indicating or display means.

12. Guidance system according to any of claims 1 to 11, wherein the glasses (20) are configured to determine and to display an operating step instruction for operating the drug delivery device (1) based on the status parameter value.

13. Guidance system according to any of claims 1 to 12, wherein the delivery device (1) is an injection device, preferably an autoinjector, and wherein the electronic module (3) is integrated inside a housing (2) of the injection device.

14. A computer program product comprising instructions which, when executed by a computing device in wearable glasses (20), cause the computing device to carry out the steps of
a. Establishing a data communication between the wearable glasses (20) and an electronic module (3) integrated in or releasably attached to a drug delivery device (1);
b. Receiving a delivery status parameter value from the electronic module (3);
c. Generating display data including status information for a user based on the received status parameter value;
d. Providing the display data to display means of the wearable glasses (20) to display the status information in an overlay (24) in a user field of view of the wearable glasses (20).

15. Method for guiding through a delivery process with a drug delivery device (1), the method comprising the steps of
a) Sensing and obtaining, by an electronic module (3) integrated in or releasably attached to the drug delivery device (1), a delivery status parameter value;
b) Transmitting, by the electronic module (3), the obtained status parameter value to wearable glasses (20);
c) Processing, by a computing device in the glasses (20), the status parameter value and generating status information for a user based on the status parameter value;
d) Displaying, by the glasses (20), the process information in an overlay (24) of a user field of view of the wearable glasses (20).
